# EUROPEAN PATENT SPECIFICATION

(11) **EP 0 747 344 B1**
(45) Date of publication and mention of the grant of the patent: **14.01.1998**
(21) Application number: 96304106.6
(22) Date of filing: 05.06.1996
(51) Int. Cl.: C07C 231/24, C07C 237/46

(54) **Process for the purification and crystallisation of iopamidol**
Verfahren zur Reinigung und Krystallisierung von Iopamidol
Procédé pour la purification et la crystallisation de l'Iopamidol

(30) Priority: 08.06.1995 PT 10172095
(43) Date of publication of application: 11.12.1996
(73) Proprietor: HOVIONE INTER LTD., 6000 Lucerne 7 (CH)
(72) Inventor: Heggie, William, Dr., Cabanas, 2950 Palmela (PT); De Mouro vaz Azevedo Mendes, Zita Maria, 1600 Lisboa (PT); De Lacerda e Oliveira Santos, Pedro Paulo, 2745 Queluz (PT)
(74) Representative: Wain, Christopher Paul

(56) References cited:
- EP-A- 0 357 467
- WO-A-95/04031
- FR-A- 2 293 919
- GB-A- 2 287 024

## Description

It has been shown that non-ionic X-ray contrast media, such as Iopamidol, present advantages in relation to prior art ionic compounds. All of these products depend on the presence of large quantities of iodine in the molecule to provide an opaque background, allowing their use to visualise internal organs. However, the quantities needed for this purpose are extremely high, being many times higher than those needed for compounds used as medicines. It is normal to inject between 100 g and 200 g of contrast media agent into a patient. For this reason, the above mentioned substances should be of very high purity, have extremely low toxicity and a minimal interference with the normal organ functions. Therefore, there is an increasing need to develop manufacturing processes for the production of contrast media giving product of the highest purity.

There exist several methods which can be used to produce high purity bulk pharmaceutical products. Possibly the most efficient method for the production of high purity product is a chromatographic process. However, in the present instance, such a method is difficult to implement given the extremely large quantities that are necessary. Chromatographic methods are usually associated with low volume and high value product.

Crystallisation methods are the most suitable but they do not always allow the successful removal of impurities with molecular structures that are very similar to that of the primary product. In the case of Iopamidol, for example, one of the most problematic impurities is that formed by the loss of one of the iodine atoms bonded to the central benzene ring. In terms of the molecular configuration in the crystal, the properties of this substance are very similar to those of Iopamidol, being easily introduced in the crystalline structure with the consequent difficulty in its removal.

Another general consideration is the fact that the molecules used as X-ray contrast media are extremely soluble, especially in aqueous media, and hence their difficult crystallisation. It is believed that this is due to the high degree of freedom associated with the hydrophilic side chains containing alcohol functions.

The prior art cites two solvent systems considered adequate for the crystallisation of Iopamidol. The first of these systems comprises the use of ethanol and/or aqueous ethanol, as described in US-A-4 001 323. The crystallisation is usually carried out by dissolution of Iopamidol in the minimum amount of water, addition of ethanol, followed by distillation of the excess water until an adequate ethanol:water ratio, usually of the order of 95:5, is obtained, after which purified Iopamidol separates. A disadvantage of this method is that the product tends to retain a certain amount of ethanol in the crystal structure, which can only be removed by drying at high temperatures under vacuum.

PCT Patent Application N° 94/02415 (publication number WO95/04031) claims an improvement in relation to the use of ethanol. Butyl alcohols, especially 2-butanol, are used in a manner similar to that mentioned above for ethanol. It is claimed that butyl alcohols are more easily removed than ethanol from the final product in the drying step.

However, these two solvent systems present the same disadvantage when used at a large scale, namely the separation of the product is too rapid, even at high temperature, causing the crystallisation process to pass through an intermediate phase in which there is formation of a pasty product which only solidifies after an extended stirring period at a reduced temperature. It is well known that the formation of this type of pasty product can give rise to the occlusion of impurities, which, otherwise, would have remained in the mother liquors of the crystallisation, and hence the final bulk product may not have the high purity level desired. There can also be losses of product which sticks to the walls, to the stirrer, etc. of the reactor where the crystallisation takes place. This situation is obviously undesirable during routine production.

It has been unexpectedly found that the use of propanol and isopropanol solves these problems. When propyl alcohols are used, the crystallisation process is much slower than with ethanol or the butyl alcohols, thereby eliminating the tendency to form pastes. Crude solid Iopamidol is dissolved in water, or if the aqueous solution is obtained directly from the reaction in which the Iopamidol is formed, any nonaqueous solvents being previously removed by conventional methods, then propanol or isopropanol is added and the ratio of propanol or isopropanol to water is corrected by means of the azeotropic properties of propanol or isopropanol with water. Alternatively, di-isopropyl ether can be used so as to obtain a ternary azeotropic system in order to attain more quickly the desired ratio of propanol or isopropanol and water. The percentage of water in the final solvent mixture is usually between 1% and 15% in relation to the propanol or isopropanol. After cooling, during which efficient stirring is maintained, Iopamidol is isolated from the solution in a well defined crystalline form.

A further advantage of this method is the fact that the so formed Iopamidol can be more easily filtered than any of the products resulting from the crystallisation with ethanol or the butyl alcohols. From an industrial point of view, this fact has obvious advantages.

Residual quantities of propanol, or isopropanol, are also easily removed by conventional drying procedures. Therefore, the use of either propanol or isopropanol to carry out the purification of Iopamidol provides a practical industrial scale process.

The table appearing hereinbelow summarises the results obtained and establishes a comparison between 2-butanol, 1-propanol and 2-propanol.

| **Comparative crystallisations of Iopamidol** | | | | | |
|---|---|---|---|---|---|
| Solvent | Paste formation | Time to beginning of crystallisation | Time of filtration | Yield (%) | % Purity (by HPLC) |
| 2-BuOH | YES | -- | 4' | 99.2 | 99.80 |
| 1-PrOH | NO | 2' | 3'30" | 99.1 | 99.84 |
| 2-PrOH | NO | 5' | 3' | 99.3 | 99.84 |
| 2-PrOH | NO | 3' | 3'30" | 98.9 | 99.86 |

The tests were carried out at a 30 g scale and the filtration time relates to tests carried out in identical equipment, under the same conditions.

The following examples serve to illustrate the present invention and are not, in any way, to be considered as a limitation thereof.

### EXAMPLE 1

Crude, crystalline and desalinated Iopamidol (30 g), resulting from the normal production process, was dissolved in water (30 ml) and concentrated to a volume of 17.5 ml. After heating to 75°-80°C, 1-propanol (212 ml), previously heated to 75°-80°C, was added and the mixture refluxed for 30 minutes, during which crystallisation occurred. A mixture of propanol/water (12 ml) was removed by azeotropic distillation at atmospheric pressure, with the concentration of water in the final mixture being 3.3%. After reflux for 30 minutes, cooling to room temperature and stirring for 3 hours, the product was filtered and dried at 60°C. The yield of Iopamidol was 29.4 g.

### EXAMPLE 2

Crude, crystalline and desalinated Iopamidol (30 g) was dissolved in water (30 ml) and concentrated to a volume of 17.5 ml. After heating to 75°-80°C, 2-propanol (230 ml), previously heated to 75°-80°C, was added and the mixture refluxed for 30 minutes, during which crystallisation occurred. A mixture of propanol/water (30 ml) was removed by azeotropic distillation at atmospheric pressure, with the concentration of water in the final mixture being 3.8%. After reflux for 30 minutes, cooling to room temperature and stirring for 3 hours, the product was filtered and dried at 60°C. The yield of Iopamidol was 29.5 g.

### EXAMPLE 3

An aqueous and desalinated solution of crude Iopamidol (containing 30.1 g of product) resulting from the normal production process, was concentrated to a volume of 17.5 ml. After heating to 75°-80°C, 1-propanol (225 ml), previously heated to 75°-80°C, was added and the mixture refluxed for 30 minutes, during which crystallisation occurred. A mixture of propanol/water (25 ml) was removed by azeotropic distillation at atmospheric pressure, with the concentration of water in the final mixture being 2.6%. After reflux for 30 minutes, cooling to room temperature and stirring for 3 hours, the product was filtered and dried at 60°C. The yield of Iopamidol was 29.8 g.

### EXAMPLE 4

An aqueous and desalinated solution of crude Iopamidol (containing 29.4 g of product) was concentrated to a volume of 17.5 ml. After heating to 75°-80°C, 2-propanol (240 ml), previously heated to 75°-80°C, was added and the mixture refluxed for 30 minutes, during which crystallisation occurred. A mixture of propanol/water (40 ml) was removed by azeotropic distillation at atmospheric pressure, with the concentration of water in the final mixture being 3.9%. After reflux for 30 minutes, cooling to room temperature and stirring for 3 hours, the product was filtered and dried at 60°C. The yield of Iopamidol was 29.1 g.

### EXAMPLE 5

Crude, crystalline and desalinated Iopamidol (5 g) was dissolved in water (5 ml). After heating to 68°-72°C, 2-propanol (40 ml), previously heated to 68-72°C, was added and the mixture refluxed for 60 minutes, with crystallisation occurring after a 30 minute period. After cooling to 0°-5°C and stirring for 30 minutes, the product was filtered, washed with 5 ml of 2-propanol and dried at 60°C. The yield of Iopamidol was 4.1 g.

### EXAMPLE 6

An aqueous and desalinated solution of crude Iopamidol (containing 30 g of product), resulting from the normal production process, was concentrated to a volume of 20 ml. After heating to 75°-80°C, 1-propanol (270 ml), previously heated to 75°-80°C, was added and the mixture refluxed for 30 minutes, during which crystallisation occurred. After addition of di-isopropyl ether (60 ml), a mixture of 1-propanol/di-isopropyl ether/water (90 ml) was removed by azeotropic distillation at atmospheric pressure, with the concentration of water in the final mixture being 3.1%. After reflux for 30 minutes, cooling to room temperature and stirring for 3 hours, the product was filtered and dried at 60°C. The yield of Iopamidol was 29.9 g.

### EXAMPLE 7

An aqueous and desalinated solution of crude Iopamidol (containing 30 g of product) was concentrated to a volume of 20 ml. After heating to 75°-80°C, 2-propanol (270 ml), previously heated to 75°-80°C, was added and the mixture refluxed for 30 minutes, during which crystallisation occurred. After addition of di-isopropyl ether (90 ml), a mixture of 2-propanol/di-isopropyl ether/water (90 ml) was removed by azeotropic distillation at atmospheric pressure, with the concentration of water in the final mixture being 3.9%. After reflux for 30 minutes, cooling to room temperature and stirring for 3 hours, the product was filtered and dried at 60°C. The yield of Iopamidol was 29.9 g.

### EXAMPLE 8

Crude, crystalline and desalinated Iopamidol (30 g) was dissolved in water (30 ml) and concentrated to a volume of 20 ml. After heating to 75°-80°C, 2-propanol (270 ml), previously heated to 75°-80°C, was added and the mixture refluxed for 30 minutes, during which crystallisation occurred. A mixture of propanol/water (90 ml) was removed by azeotropic distillation at atmospheric pressure, the concentration of water in the final mixture being 5.0%. After reflux for 30 minutes, cooling to room temperature and stirring for 3 hours, the product was filtered and dried at 60°C. The yield of Iopamidol was 29.6 g.

## Claims

1. Process for the purification and crystallisation of Iopamidol, characterised by the fact that propanol is added to an aqueous solution of Iopamidol, obtained either by the dissolution of Iopamidol in water or directly from the reaction in which the Iopamidol is formed, followed, if necessary, by azeotropic distillation to reduce the amount of water with respect to the amount of propanol.

2. Process according to claim 1, characterised by the fact that the propanol is 1-propanol.

3. Process according to claim 1, characterised by the fact that the propanol is 2-propanol.

4. Process according to claim 1, characterised by the fact that the starting material is crude Iopamidol, in the solid form.

5. Process according to claim 1, characterised by the fact that the starting material is crude Iopamidol, in aqueous solution.

6. Process according to any of claims 4 or 5, characterised by the fact that propanol is added to an aqueous solution of Iopamidol, followed, if necessary, by azeotropic distillation to reduce the amount of water with respect to the amount of propanol.

7. Process according to any of claims 4 or 5, characterised by the fact that the quantity of propanol is comprised between 5 and 15 times, in volume, with respect to the weight of the Iopamidol present.

8. Process according to any of claims 4 or 5, characterised by the fact that the ratio of water to propanol is comprised between 1:99 and 15:85.

9. Process according to claim 8, characterised by the fact that the ratio of water to propanol is established by the use of a binary azeotropic distillation, the two components being water and propanol.

10. Process according to claim 8, characterised by the fact that the ratio of water to propanol is established by the use of a ternary azeotropic distillation, the three components being water, di-isopropyl ether and propanol.

## Patentansprüche

1. Verfahren zur Reinigung und Kristallisation von Iopamidol, dadurch gekennzeichnet, daß Propanol zu einer wäßrigen Lösung von Iopamidol gegeben wird, die entweder durch Auflösen von Iopamidol in Wasser oder direkt aus der Reaktion, bei der das Iopamidol gebildet wird, erhalten wird, wobei anschließend, falls erforderlich, eine azeotrope Destillation erfolgt, um die Menge des Wassers in bezug auf die Menge des Propanols zu reduzieren.

2. Verfahren gemäß Anspruch 1, dadurch gekennzeichnet, daß es sich bei dem Propanol um 1-Propanol handelt.

3. Verfahren gemäß Anspruch 1, dadurch gekennzeichnet, daß es sich bei dem Propanol um 2-Propanol handelt.

4. Verfahren gemäß Anspruch 1, dadurch gekennzeichnet, daß es sich bei dem Ausgangsmaterial um rohes Iopamidol in fester Form handelt.

5. Verfahren gemäß Anspruch 1, dadurch gekennzeichnet, daß es sich bei dem Ausgangsmaterial um rohes Iopamidol in wäßriger Lösung handelt.

6. Verfahren gemäß einem der Ansprüche 4 oder 5, dadurch gekennzeichnet, daß Propanol zu einer wäßrigen Lösung von Iopamidol gegeben wird, wobei anschließend, falls erforderlich, eine azeotrope Destillation erfolgt, um die Menge des Wassers in bezug auf die Menge des Propanols zu reduzieren.

7. Verfahren gemäß einem der Ansprüche 4 oder 5, dadurch gekennzeichnet, daß die Menge des Propanols als Volumen zwischen 5- und 15mal so groß ist wie das Gewicht des vorhandenen Iopamidols.

8. Verfahren gemäß einem der Ansprüche 4 oder 5, dadurch gekennzeichnet, daß das Verhältnis von Wasser zu Propanol zwischen 1:99 und 15:85 liegt.

9. Verfahren gemäß Anspruch 8, dadurch gekennzeichnet, daß das Verhältnis von Wasser zu Propanol durch die Verwendung einer binären azeotropen Destillation erreicht wird, wobei die beiden Komponenten Wasser und Propanol sind.

10. Verfahren gemäß Anspruch 8, dadurch gekennzeichnet, daß das Verhältnis von Wasser zu Propanol durch die Verwendung einer ternären azeotropen Destillation erreicht wird, wobei die drei Komponenten Wasser, Diisopropylether und Propanol sind.

## Revendications

1. Procédé pour la purification et la cristallisation d'iopamidol, caractérisé en ce que du propanol est ajouté à une solution aqueuse d'iopamidol, obtenue soit par la dissolution d'iopamidol dans de l'eau soit directement de la réaction dans laquelle l'iopamidol est formé, opération suivie, suivant les nécessités, par une distillation azéotropique pour réduire la quantité d'eau par rapport à la quantité de propanol.

2. Procédé suivant la revendication 1, caractérisé en ce que le propanol est le 1-propanol.

3. Procédé suivant la revendication 1, caractérisé en ce que le propanol est le 2-propanol.

4. Procédé suivant la revendication 1, caractérisé en ce que la matière de départ est de l'iopamidol brut, sous la forme solide.

5. Procédé suivant la revendication 1, caractérisé en ce que la matière de départ est de l'iopamidol brut, en solution aqueuse.

6. Procédé suivant l'une ou l'autre des revendications 4 et 5, caractérisé en ce que du propanol est ajouté à une solution aqueuse d'iopamidol, opération suivie, suivant les nécessités, d'une distillation azéotropique pour réduire la quantité d'eau par rapport à la quantité de propanol.

7. Procédé suivant l'une ou l'autre des revendications 4 et 5, caractérisé en ce que la quantité de propanol est comprise entre 5 et 15 fois, en volume, par rapport au poids de l'iopamidol présent.

8. Procédé suivant l'une ou l'autre des revendications 4 et 5, caractérisé en ce que le rapport de l'eau au propanol est compris entre 1/99 et 15/85.

9. Procédé suivant la revendication 8, caractérisé en ce que le rapport de l'eau au propanol est établi par l'utilisation d'une distillation azéotropique binaire, les deux composants étant l'eau et le propanol.

10. Procédé suivant la revendication 8, caractérisé en ce que le rapport de l'eau au propanol est établi par l'utilisation d'une distillation azéotropique ternaire, les trois composants étant l'eau, l'éther diisopropylique et le propanol.
